# EUROPÄISCHE PATENTSCHRIFT

(11) **EP 0 527 704 B1**
(45) Veröffentlichungstag und Bekanntmachung des Hinweises auf die Patenterteilung: **28.08.1996**
(21) Anmeldenummer: 92810585.7
(22) Anmeldetag: 31.07.1992
(51) Int. Cl.: C09B 29/33, D06P 3/24, D06P 1/06, C09B 29/09, C09B 31/11, C07C 309/17, C07C 309/14

(54) **Farbstoffe, Verfahren zu deren Herstellung und deren Verwendung**
Dyestuffs, process for their preparation and the use thereof
Colorants, leur procédé de préparation et leur utilisation

(30) Priorität: 08.08.1991 CH 2344/91
(43) Veröffentlichungstag der Anmeldung: 17.02.1993
(73) Patentinhaber: CIBA-GEIGY AG, 4002 Basel (CH)
(72) Erfinder: Adam, Jean-Marie, Dr., F-68300 Rosenau (FR); Eichenberger, Thomas, Dr., CH-4056 Basel (CH)

(56) Entgegenhaltungen:
- EP-A- 0 183 151
- EP-A- 0 311 949
- FR-A- 2 294 263
- FR-A- 2 378 003
- FR-A- 2 401 197

## Beschreibung

Die vorliegende Erfindung betrifft neue Farbstoffe, Verfahren zu deren Herstellung und Verwendung dieser Farbstoffe zum Färben und Bedrucken von Fasermaterialien, insbesondere textilen Fasermaterialien.

Gegenstand der vorliegenden Erfindung sind Farbstoffe der Formel 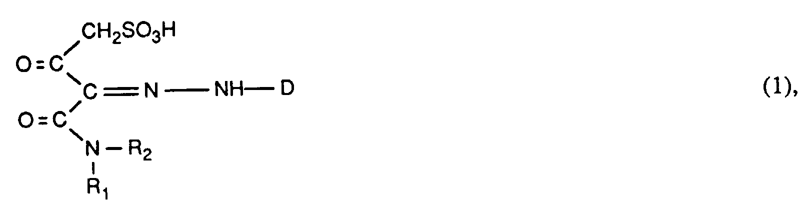 worin D der Rest einer Diazokomponente der Benzol- oder Naphthalinreihe oder der heterocyclischen Reihe ist, R₁ Wasserstoff oder C₁-C₄-Alkyl und R₂ gegebenenfalls substituiertes Phenyl ist.

Der Rest D in Formel (1) kann die bei Diazokomponenten üblichen Substituenten enthalten, z.B. Alkylgruppen mit 1 bis 8, vorzugsweise 1 bis 4 Kohlenstoffatomen, wie z.B. Methyl, Aethyl, Propyl, Isopropyl, Butyl, Isobutyl, sek.-Butyl, tert.-Butyl, Pentyl, Hexyl, Heptyl oder Octyl, Alkoxygruppen mit 1 bis 8 Kohlenstoffatomen, insbesondere 1 bis 4 Kohlenstoffatomen, wie z.B. Methoxy, Aethoxy, Propoxy, Isopropoxy, Butoxy, Acylaminogruppen wie Alkanoylaminogruppen mit 2 bis 8 Kohlenstoffatomen, insbesondere 2 bis 4 Kohlenstoffatomen, und Alkoxycarbonylaminogruppen mit 2 bis 8 Kohlenstoffatomen, wie z.B. Acetylamino, Propionylamino, Methoxycarbonylamino, Aethoxycarbonylamino, Alkanoylgruppen mit 2 bis 8, vorzugsweise 2 bis 4 Kohlenstoffatomen, wie z.B. Acetyl, Propionyl, Butyryl oder Isobutyryl, Amino, Mono- oder Dialkylamino mit 1 bis 8 Kohlenstoffatomen im Alkylrest, Carboxy, Alkoxycarbonyl mit 1 bis 8 Kohlenstoffatomen im Alkoxyrest, Halogen, wie Fluor, Brom oder insbesondere Chlor, Sulfo, Arylazogruppen wie z.B. die Phenylazo- und Naphthylazogruppe, heterocyclische Ringsysteme, wie z.B. Benzo[b]furyl, Benzo[b]thiophenyl, Indolyl, Benzoxazolyl, Benzothiazolyl, Benzimidazolyl oder Benzotriazolyl, wobei die angegebenen Phenyl- und Naphthylreste sowie die angegebenen heterocyclischen Ringsysteme durch die oben angegebenen Substituenten weitersubstituiert sein können. Der Rest D in Formel (1) ist vorzugsweise der Rest einer Diazokomponente der Benzolreihe.

Bedeutet R₁ C₁-C₄-Alkyl, so handelt es sich um Methyl, Äthyl, Propyl, Isopropyl, Butyl, sek.-Butyl, Isobutyl oder tert.-Butyl.

Als Phenyl kommt für R₂ in Formel (1) ein unsubstituierter oder ein durch C₁-C₈-Alkyl, insbesondere C₁-C₄-Alkyl, wie z.B. Methyl, Aethyl, Propyl, Isopropyl, Butyl, Isobutyl, sek.-Butyl und tert.-Butyl, C₁-C₄-Alkoxy, wie Methoxy, Aethoxy, Propoxy, Isopropoxy, Butoxy, Isobutoxy und sek.-Butoxy, Halogen, wie Fluor, Chlor und Brom, C₂-C₄-Alkanoylamino, wie Acetylamino und Propionylamino, und Sulfo substituierter Phenylrest in Betracht.

Bevorzugt sind Farbstoffe der Formel (1), worin R₁ Wasserstoff ist.

Ebenfalls bevorzugt sind Farbstoffe der Formel (1), worin R₂ gegebenenfalls durch C₁-C₄-Alkyl, C₁-C₄-Alkoxy, C₂-C₄-Alkanoylamino, Halogen oder Sulfo, insbesondere durch C₁-C₄-Alkyl, C₁-C₄-Alkoxy oder Halogen, substituiertes Phenyl ist.

Die Farbstoffe der Formel (1) enthalten vorzugsweise nur eine Sulfogruppe

Weiterhin bevorzugt sind Farbstoffe der Formel (1), worin D ein Rest der Formel 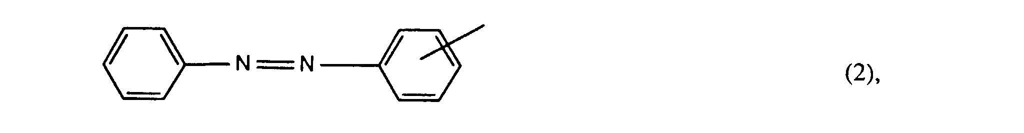 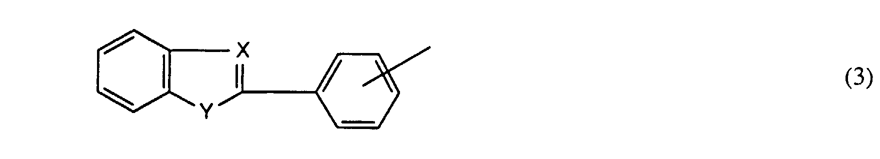 oder 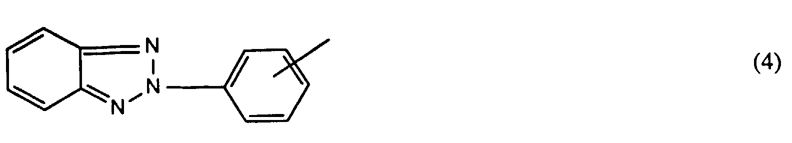 ist, wobei die Reste der Formeln (2), (3) und (4) gegebenenfalls wie unter Formel (1) angegeben substituiert sind und im Rest der Formel (3) X ein Stickstoffatom oder die CH-Gruppe ist und Y ein Sauerstoffatom, Schwefelatom oder die NH-Gruppe bedeutet. Vorzugsweise sind die Reste D gegebenenfalls durch C₁-C₄-Alkyl, C₁-C₄-Alkoxy, Halogen, C₂-C₄-Alkanoylamino oder Sulfo, insbesondere durch C₁-C₄-Alkyl, C₁-C₄-Alkoxy, C₂-C₄-Alkanoylamino oder Halogen, substituiert.

Besonders bevorzugt sind Farbstoffe der Formel (1), worin R₁ Wasserstoff, R₂ gegebenenfalls durch C₁-C₄-Alkyl, C₁-C₄-Alkoxy, C₂-C₄-Alkanoylamino, Halogen oder Sulfo substituiertes Phenyl ist und D ein Rest der Formel (2), (3) oder (4) ist, wobei für die Reste der Formeln (2), (3) und (4) die oben angegebenen Bedeutungen und Bevorzugungen gelten. Ganz besonders bevorzugt sind Farbstoffe der Formel 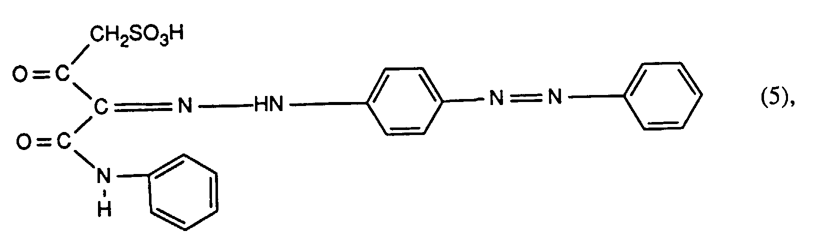 sowie Farbstoffe der Formeln 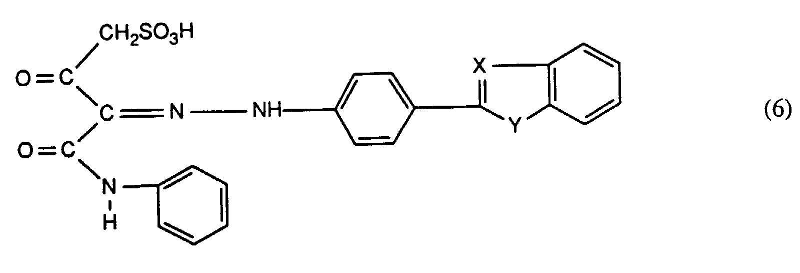 und 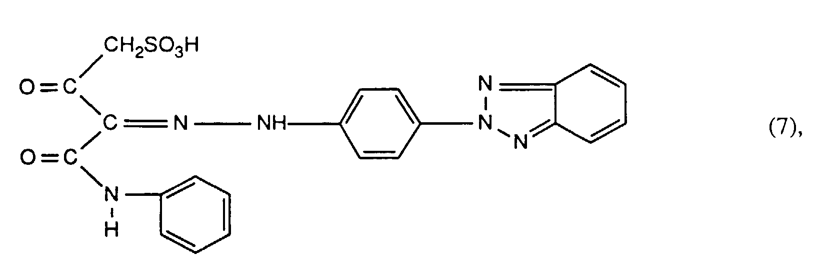 wobei die Farbstoffe der Formeln (5), (6) und (7) gegebenenfalls durch C₁-C₄-Alkyl, C₁-C₄-Alkoxy, C₂-C₄-Alkanoylamino, Halogen oder Sulfo, insbesondere durch C₁-C₄-Alkyl, C₁-C₄-Alkoxy, C₂-C₄-Alkanoylamino oder Halogen, substituiert sind und im Farbstoff der Formel (6) X ein Stickstoffatom oder die CH-Gruppe ist und Y ein Sauerstoffatom, Schwefelatom oder die NH-Gruppe bedeutet.

Gegenstand der Erfindung ist ferner ein Verfahren zur Herstellung der Farbstoffe der Formel (1), welches dadurch gekennzeichnet ist, dass man ein Amin der Formel

D-NH₂ (8)

diazotiert und auf eine Kupplungskomponente der Formel 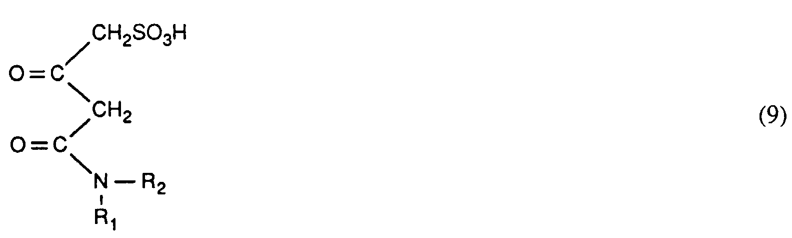 kuppelt, wobei D, R₁ und R₂ die unter Formel (1) angegebenen Bedeutungen haben.

Die Diazotierung des Amins der Formel (8) erfolgt in an sich bekannter Weise, z.B. mit einem Nitrit, z.B. mit einem Alkalimetallnitrit, wie Natriumnitrit, in einem mineralsauren Medium, z.B. in einem salzsauren Medium, bei Temperaturen von beispielsweise -5 bis 30°C und vorzugsweise bei 0 bis 10°C.

Die Kupplung auf die Kupplungskomponente der Formel (9) erfolgt in an sich bekannter Weise, bei sauren, neutralen bis alkalischen pH-Werten und Temperaturen von beispielsweise 0 bis 30°C.

Als Beispiele für Amine der Formel (8) seien 4-Aminoazobenzol,
3-Methoxy-4-aminoazobenzol, 2-(4′-Aminophenyl)-benzothiazol,
2-(4′-Aminophenyl)-6-methyl-benzothiazol, 2-(4′-Aminophenyl)-benzoxazol,
2-(4′-Aminophenyl)-5-methyl-benzoxazol, 2-(4′-Aminophenyl)-benzimidazol,
2-(4′-Aminophenyl)-benzotriazol, 2-(4′-Aminophenyl)-benzo[b]furan und
2-(4′-Aminophenyl)-5-methyl-benzo[b]furan genannt.

Als Beispiele für Kupplungskomponenten der Formel (9) seien Verbindungen der Formel (9) genannt, worin R₁ Wasserstoff ist und R₂ Phenyl, 2- oder 4-Methoxyphenyl, 2,4-Dimethoxyphenyl, 2,4-Diäthoxyphenyl, 2,4-Dimethoxy-5-chlorophenyl, 2,5-Dimethoxy-4-chlorophenyl, 2- oder 4-Chlorophenyl, 2,4-Dichlorophenyl oder 2-Methyl-3-chlorophenyl genannt.

Bevorzugte Ausführungsformen des erfindungsgemässen Verfahrens sind dadurch gekennzeichnet, dass man
- eine Kupplungskomponente der Formel (9) verwendet, worin R₁ Wasserstoff ist;
- eine Kupplungskomponente der Formel (9) verwendet, worin R₂ gegebenenfalls durch C₁-C₄-Alkyl, C₁-C₄-Alkoxy, C₂-C₄-Alkanoylamino, Halogen oder Sulfo, insbesondere durch C₁-C₄-Alkyl, C₁-C₄-Alkoxy oder Halogen, substituiertes Phenyl ist;
- ein Amin der Formel (8) verwendet, worin D ein Rest der Formel (2), (3) oder (4) ist, wobei die Reste der Formeln (2), (3) und (4) gegebenenfalls durch C₁-C₄-Alkyl, C₁-C₄-Alkoxy, C₂-C₄-Alkanoylamino, Halogen oder Sulfo, insbesondere durch C₁-C₄-Alkyl, C₁-C₄-Alkoxy, C₂-C₄-Alkanoylamino oder Halogen, substituiert sind und im Rest der Formel (3) X und Y die unter Formel (3) angegebenen Bedeutungen haben;
- ein Amin der Formel (8) und eine Kupplungskomponente der Formel (9) verwendet, welche zusammen nur eine Sulfogruppe enthalten.

Eine besonders bevorzugte Ausführungsform des erfindungsgemässen Verfahrens ist dadurch gekennzeichnet, dass man ein Amin der Formel 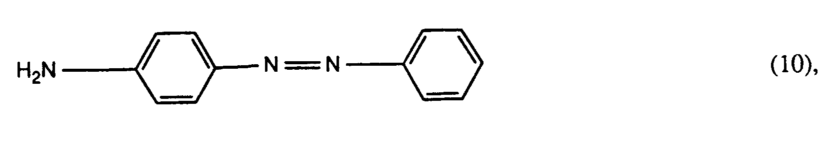 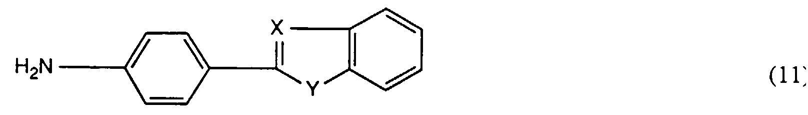 oder 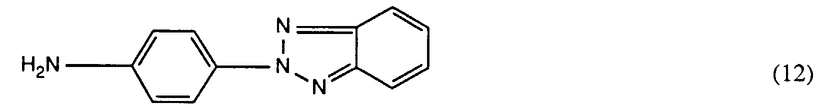 diazotiert und auf eine Kupplungskomponente der Formel 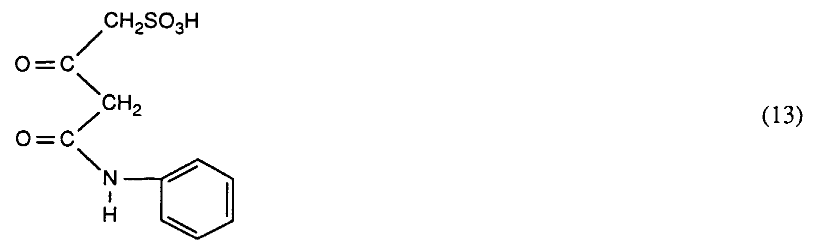 kuppelt, wobei X und Y im Amin der Formel (11) die unter Formel (3) angegebenen Bedeutungen haben und die Amine der Formeln ( 10), ( 11 ) und ( 12) und die Kupplungskomponente der Formel (13) gegebenenfalls durch C₁-C₄-Alkyl, C₁-C₄-Alkoxy, C₂-C₄-Alkanoylamino, Halogen oder Sulfo, insbesondere durch C₁-C₄-Alkyl, C₁-C₄-Alkoxy, C₂-C₄-Alkanoylamino oder Halogen, substituiert sind.

Die Amine der Formel (8) sind bekannt oder können in Analogie zu bekannten Verbindungen hergestellt werden.

Die Kupplungskomponenten der Formel (9) sind neu und stellen einen weiteren Gegenstand der vorliegenden Erfindung dar.

Gegenstand der vorliegenden Erfindung sind somit ferner Verbindungen der Formel (9), worin R₁ und R₂ die unter Formel (1) angegebenen Bedeutungen haben.

Bevorzugt sind Verbindungen der Formel (9), worin R₁ Wasserstoff ist.

Ebenfalls bevorzugt sind Verbindungen der Formel (9), worin R₂ gegebenenfalls durch C₁-C₄-Alkyl, C₁-C₄-Alkoxy, C₂-C₄-Alkanoylamino, Halogen oder Sulfo, insbesondere durch C₁-C₄-Alkyl, C₁-C₄-Alkoxy oder Halogen, substituiertes Phenyl ist.

Besonders bevorzugt sind Verbindungen der Formel (9), worin R₁ Wasserstoff ist und R₂ gegebenenfalls durch C₁-C₄-Alkyl, C₁-C₄-Alkoxy oder Halogen substituiertes Phenyl ist.

Die Verbindungen der Formel (9) werden hergestellt, indem man eine Verbindung der Formel 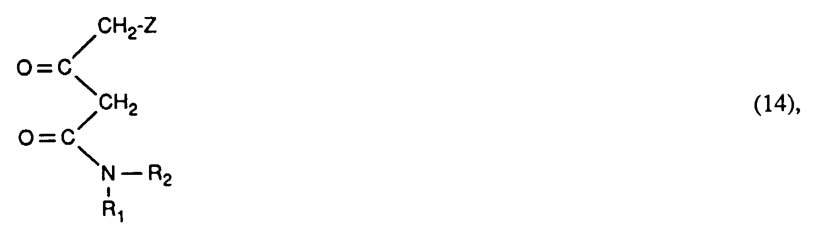 worin R₁ und R₂ die unter Formel (1) angegebenen Bedeutungen haben und Z eine anionische Abgangsgruppe ist, durch Austausch des Restes Z durch eine Sulfogruppe zur Verbindung der Formel (9) umsetzt.

Als anionische Abgangsgruppe Z kommt z.B. Halogen, wie z.B. Fluor, Brom oder insbesondere Chlor, in Betracht.

Die Einführung der Sulfogruppe zur Verbindung der Formel (9) erfolgt durch Austausch des Restes Z durch eine Sulfogruppe, wie z.B. mit Alkalisulfit, insbesondere Natriumsulfit, in einem Lösungsmittel, wie z.B. Wasser oder einer Wasser/Aethanol-Mischung, bei Temperaturen von z.B. 30 bis 100°C, insbesondere bei Temperaturen von 50 bis 70°C, bei saurem, neutralem bis alkalischem pH-Wert.

Die Verbindungen der Formel (14) sind bekannt oder können in Analogie zu bekannten Verbindungen hergestellt werden.

Die Farbstoffe der Formel (1) sowie die Verbindungen der Formel (9) liegen entweder in der Form ihrer freien Säure oder vorzugsweise als deren Salze vor.

Als Salze kommen beispielsweise die Alkali- oder Ammoniumsalze oder die Salze eines organischen Amins in Betracht.

Als Beispiel seien die Natrium-, Lithium-, Kalium- oder Ammoniumsalze oder das Salz des Mono-, Di- oder Triäthanolamins genannt.

Die erfindungsgemäßen Farbstoffe der Formel (1) eignen sich nach an sich bekannten Methoden zum Färben und Bedrucken von stickstoffhaltigen oder hydroxygruppenhaltigen Fasermaterialien, Papier oder Leder, wie z.B. textilen Fasermaterialien aus Cellulose, Seide und natürlichen und synthetischen Polyamidfasermaterialien. Die erfindungsgemässen Farbstoffe der Formel (1) können in allgemein üblicher, gegebenenfalls zuvor aufbereiteter Form zum Färben oder Bedrucken verwendet werden. Man erhält egale Färbungen mit guten Allgemeinechtheiten, insbesondere guter Reib-, Nass-, Nassreib- und Lichtechtheit. Ferner sind die erfindungsgemässen Farbstoffe gut wasserlöslich. Weiterhin zeigen die erfindungsgemässen Farbstoffe ein gutes Aufbauvermögen und sind gut mit anderen Farbstoffen kombinierbar. Ferner eignen sich die erfindungsgemässen Farbstoffe der Formel (1) zum Trichromie-Färben oder -Bedrucken von natürlichem und synthetischem Polyamidfasermaterial mit Farbstoffmischungen, welche einen Farbstoff der Formel (1) zusammen mit einem rotfärbenden Farbstoff und einem blaufärbenden Farbstoff enthalten. Das oben genannte Textilmaterial kann in den verschiedensten Verarbeitungsformen vorliegen, wie z.B. als Faser, Garn, Gewebe oder Gewirke.

In den folgenden Beispielen stehen Teile für Gewichtsteile. Die Temperaturen sind in Celsiusgraden angegeben. Die Beziehung zwischen Gewichtsteilen und Volumenteilen ist dieselbe wie diejenige zwischen Gramm und Kubikzentimeter.

Beispiel 1: 88 Teile Diketen werden in 660 Teilen Tetrachlorkohlenstoff gelöst und auf eine Temperatur von -20° abgekühlt. Bei dieser Temperatur werden innerhalb von einer Stunde 71 Teile Chlorgas eingeleitet, 90 Minuten bei -20° nachgerührt und die Temperatur auf 0 bis 5° erhöht. Es wird eine separate Lösung von 126 Teilen p-Anisidin und 102 Teilen Triäthylamin in 1300 Teilen Toluol hergestellt und diese Lösung auf eine Temperatur von 0 bis 5° abgekühlt. Die so hergestellte Lösung wird innerhalb von 45 Minuten in die wie eingangs beschrieben erhaltene Lösung eingerührt, wobei die Temperatur unterhalb von 5° gehalten wird. Anschliessend wird 6 Stunden bei 0 bis 5° nachgerührt. Man saugt ab und wäscht gründlich mit 900 Teilen Toluol und anschliessend mit 1000 Teilen Wasser nach. Nach Trocknung erhält man 218 Teile eines beigen Pulvers, das der Verbindung der Formel 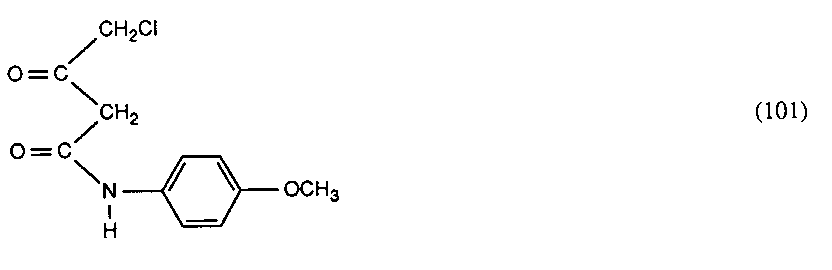 entspricht.

48 Teile der wie oben angegeben erhaltenen Verbindung der Formel (101) werden in ein Gemisch von 400 Teilen Wasser und 800 Teilen Aethanol gegeben und die Temperatur wird auf 60° erhöht. Innerhalb von 10 Minuten wird eine Lösung von 26 Teilen Natriumsulfit in 200 Teilen Wasser zugegeben und das Reakrionsgemisch noch eine Stunde gerührt, wobei die Temperatur bei 60° gehalten wird. Das Lösungsmittel wird durch Gefriertrocknen entfernt. Man erhält 74 Teile eines farblosen Pulvers, das in Form der freien Säure der Verbindung der Formel 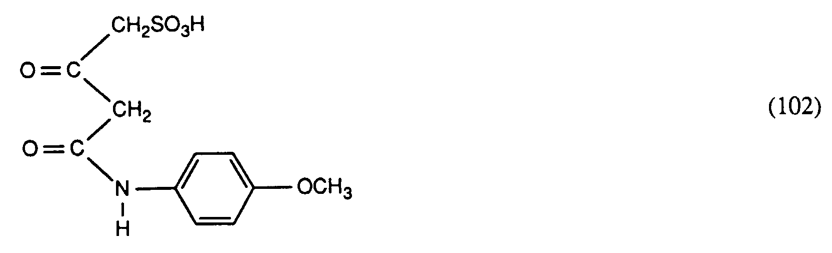 entspricht und eine Reinheit von 83% aufweist.

Beispiele 2 bis 11: Verfährt man wie in Beispiel 1 angegeben, verwendet jedoch statt 126 Teilen p-Anisidin eine äquimolare Menge einer der in Tabelle 1 in Spalte 2 angegebenen Anilinoverbindungen, so erhält man die in Tabelle 1 in Spalte 3 angegebenen Acetoacetanilidverbindungen.

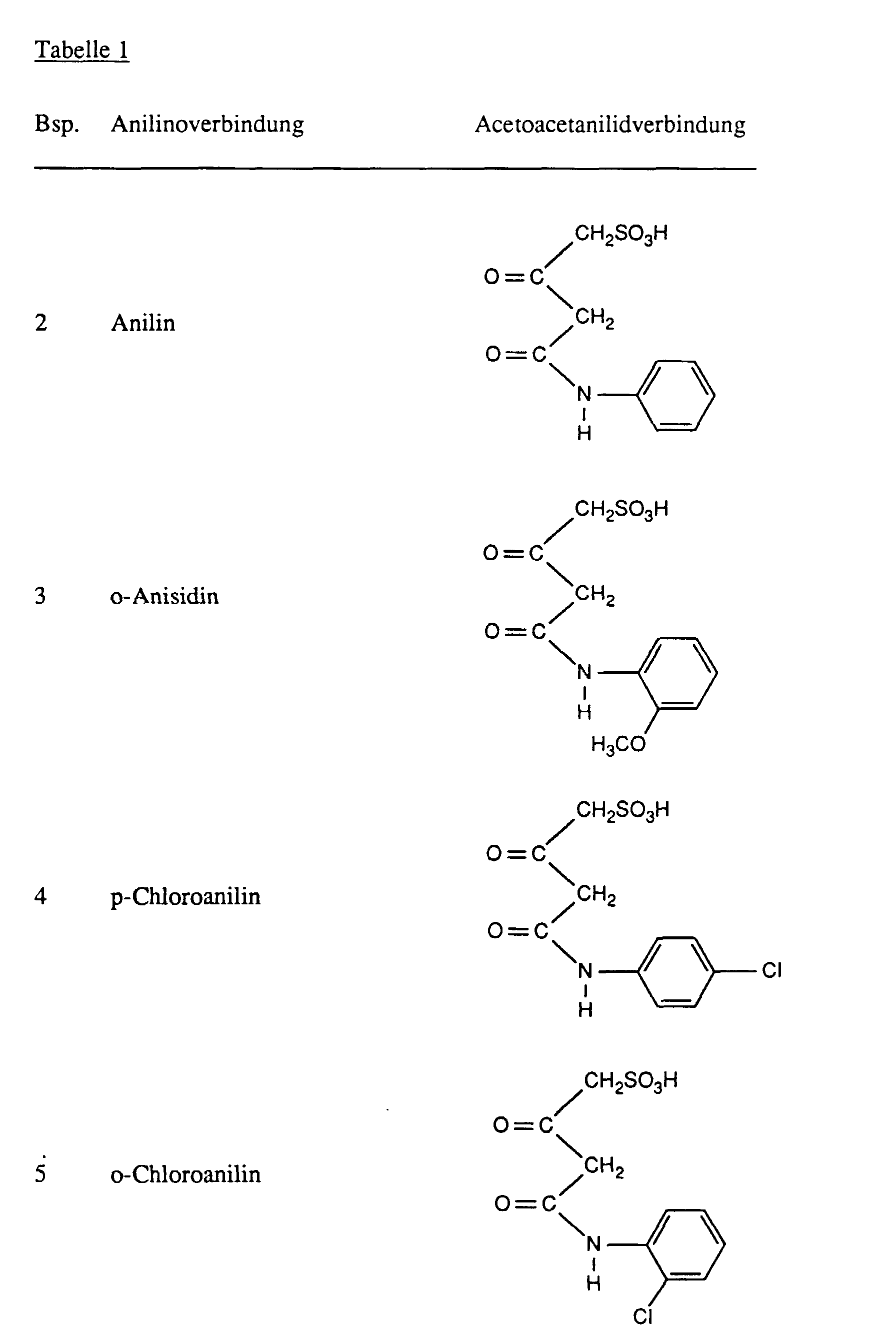

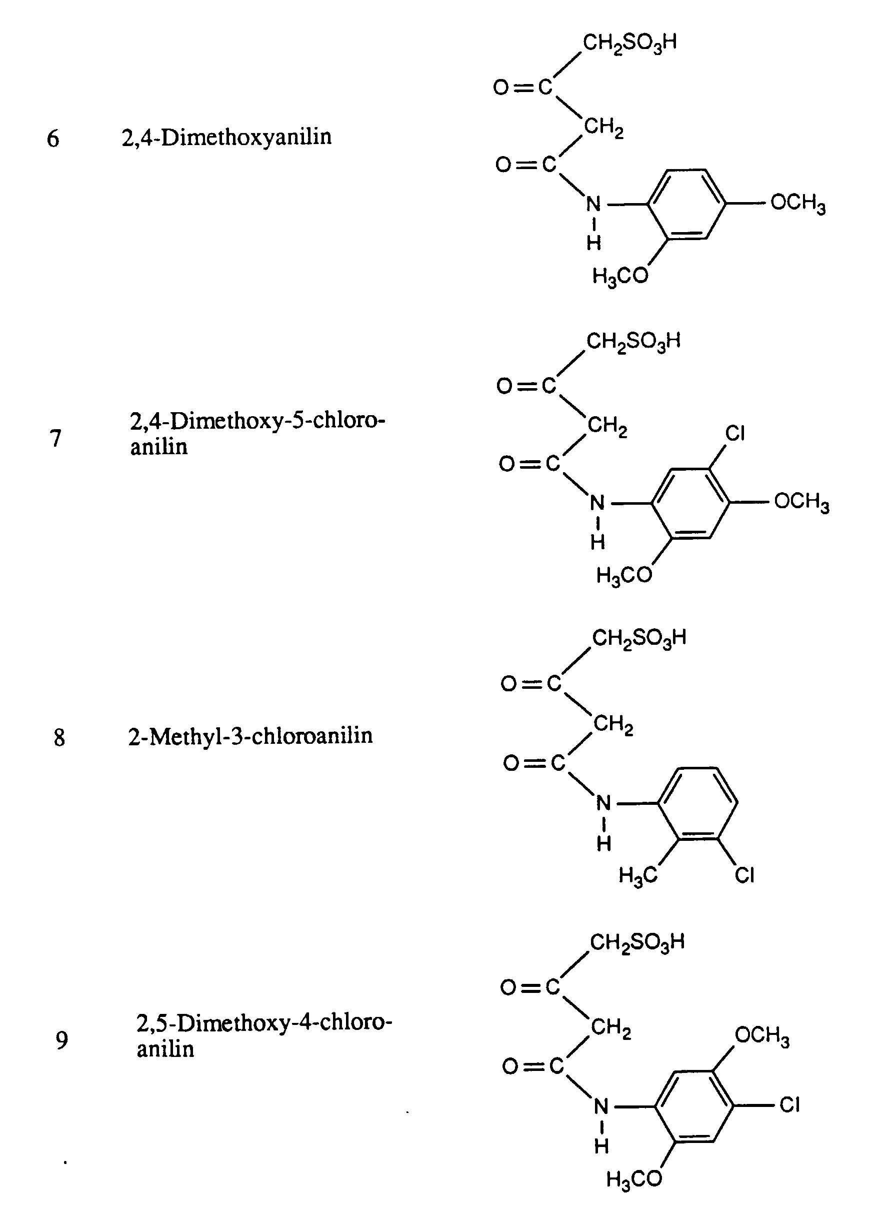

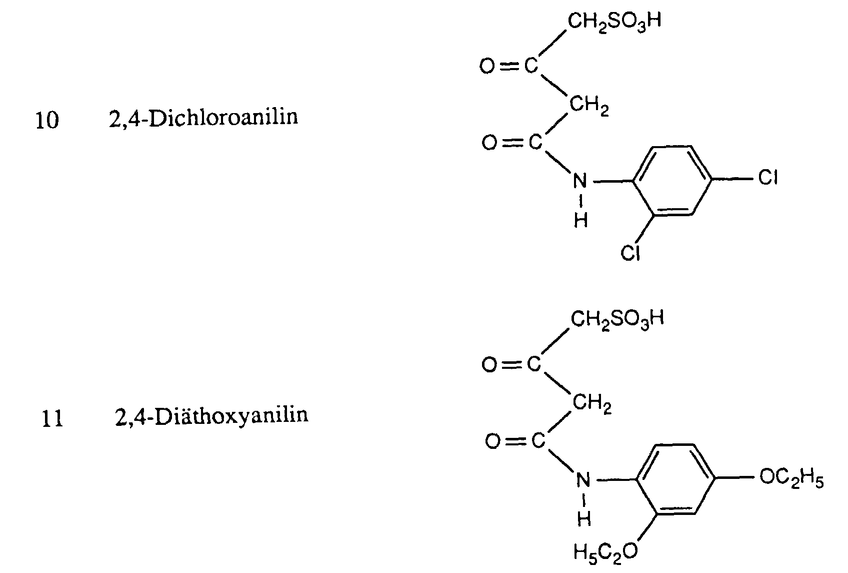

Beispiel 12: 39 Teile 4-Aminoazobenzol werden in 600 Teilen Wasser aufgerührt und durch Zugabe von 400 Teilen Eis gekühlt. Man gibt 47 Teile Salzsäure (32%) zu, hält die Temperatur durch äussere Kühlung auf 0 bis 5° und gibt innerhalb von 10 Minuten eine Lösung von 15 Teilen Natriumnitrit in 50 Teilen Wasser zu, wobei die Temperatur unterhalb von 5° gehalten wird. Die rotbraune Lösung wird noch 2 Stunden bei einer Temperatur von 0 bis 5° gerührt, und anschliessend wird mit Harnstoff ein möglicher Nitritüberschuss zerstört. Unterdessen wird eine separate Lösung von 82 Teilen der Verbindung der Formel (102) gemäss Beispiel 1 (83%) und 91 Teilen Natriumacetat in 600 Teilen Wasser bereitet und auf eine Temperatur von 0 bis 5° abgekühlt. Innerhalb von 20 Minuten wird die oben beschriebene Diazoniumsalzlösung in die letztere separat hergestellte Lösung eingerührt, wobei die Temperatur unterhalb von 5° gehalten wird. Man rührt die orange Suspension noch 5 Stunden bei einer Temperatur von 0 bis 5°, dann weitere 15 Stunden bei Raumtemperatur und saugt ab. Nach Trocknung erhält man 97 Teile eines orangen Pulvers, das im Form der freien Säure dem Farbstoff der Formel 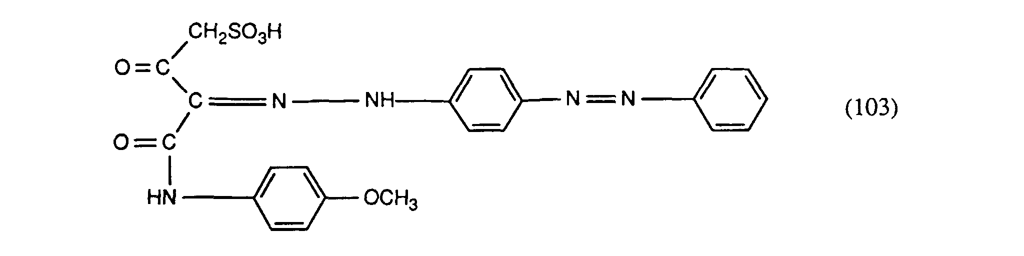 entspricht. Der Farbstoff der Formel (103) färbt natürliches und synthetisches Polyamidfasermaterial in gelben Farbtönen.

Beispiele 13 bis 34: Verfährt man wie in Beispiel 12 angegeben, verwendet jedoch gegebenenfalls statt 82 Teile der Verbindung der Formel (102) gemäss Beispiel 1 eine äquimolare Menge einer der in Tabelle 2 in Spalte 2 angegebenen Kupplungskomponenten und gegebenenfalls statt 39 Teile 4-Aminoazobenzol eine äquimolare Menge eines der in Tabelle 2 in Spalte 3 angegebenen Amine, so erhält man analoge Farbstoffe, die natürliches und synthetisches Polyamidfasermaterial in den in Spalte 4 angegebenen Farbtönen färben.

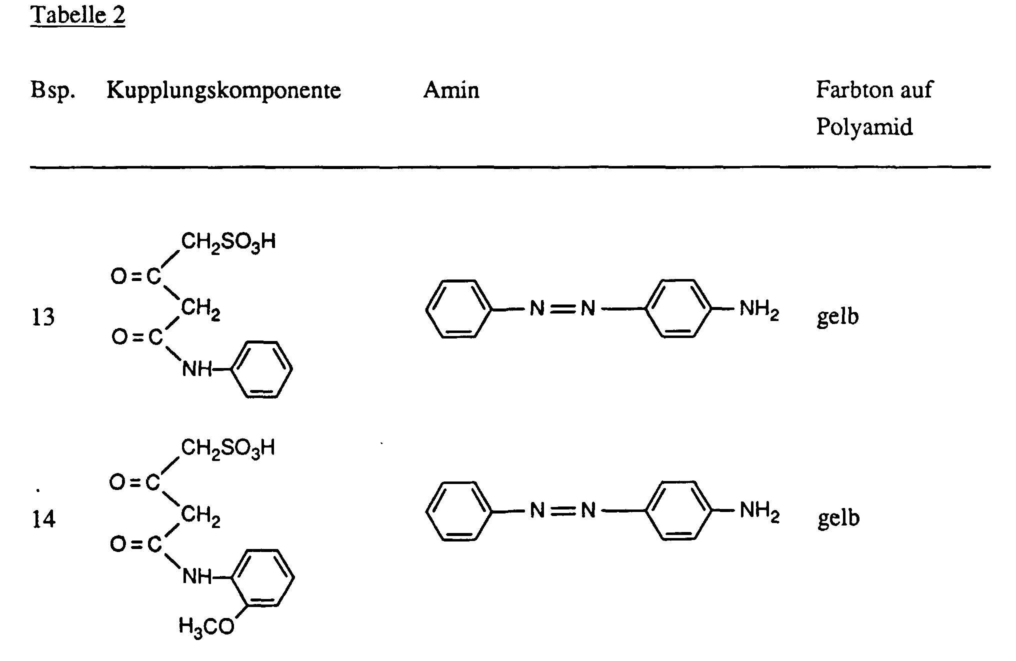

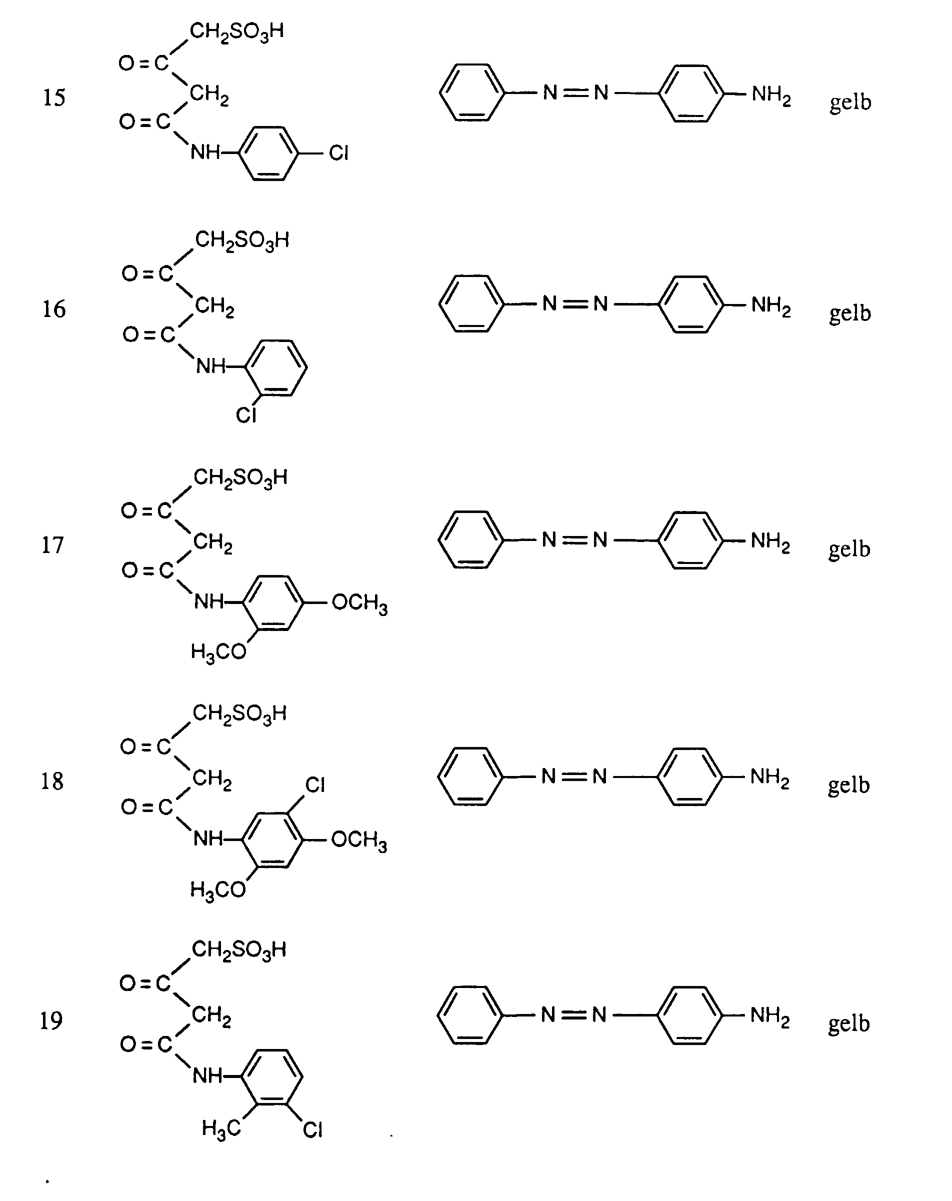

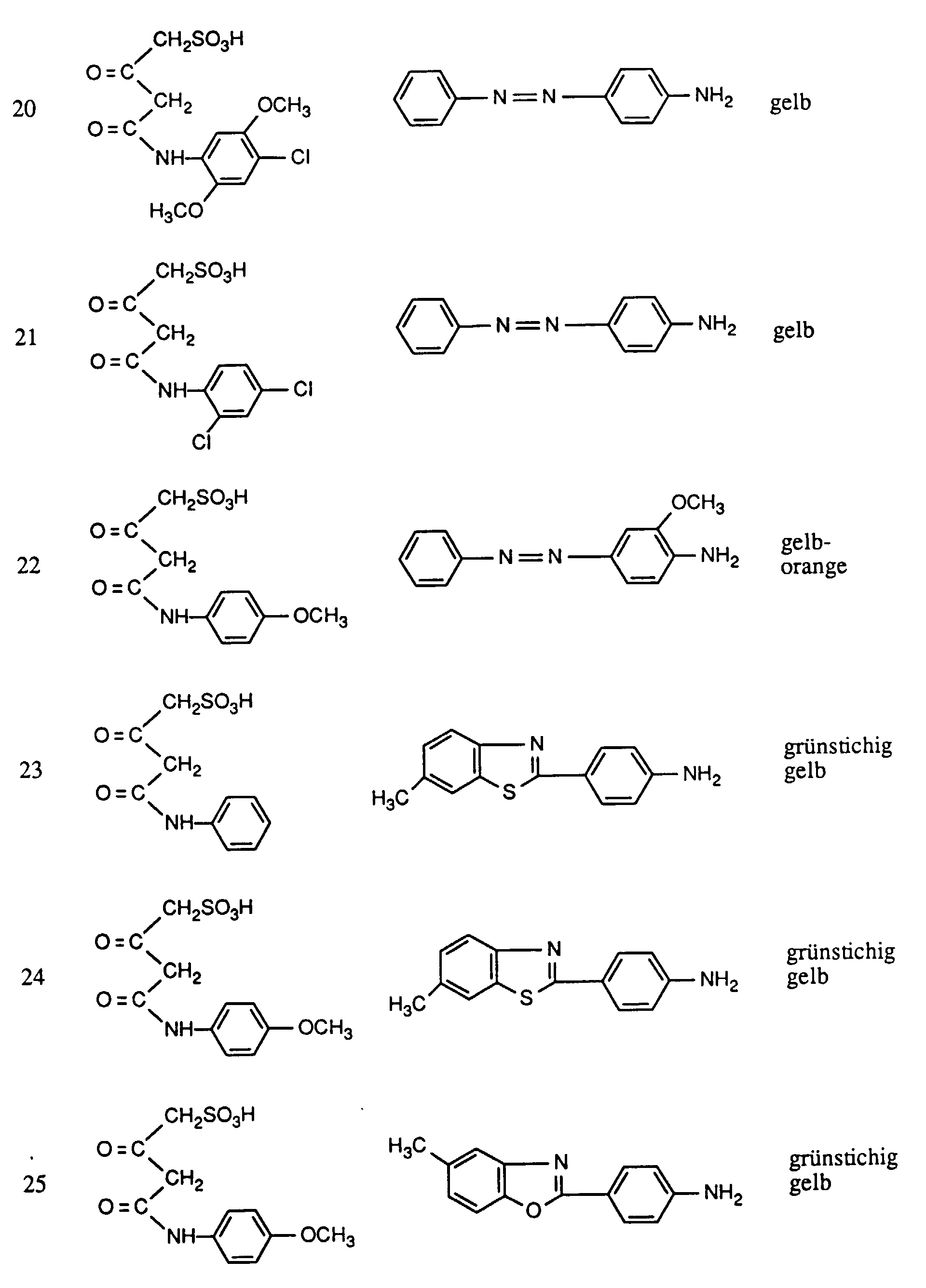

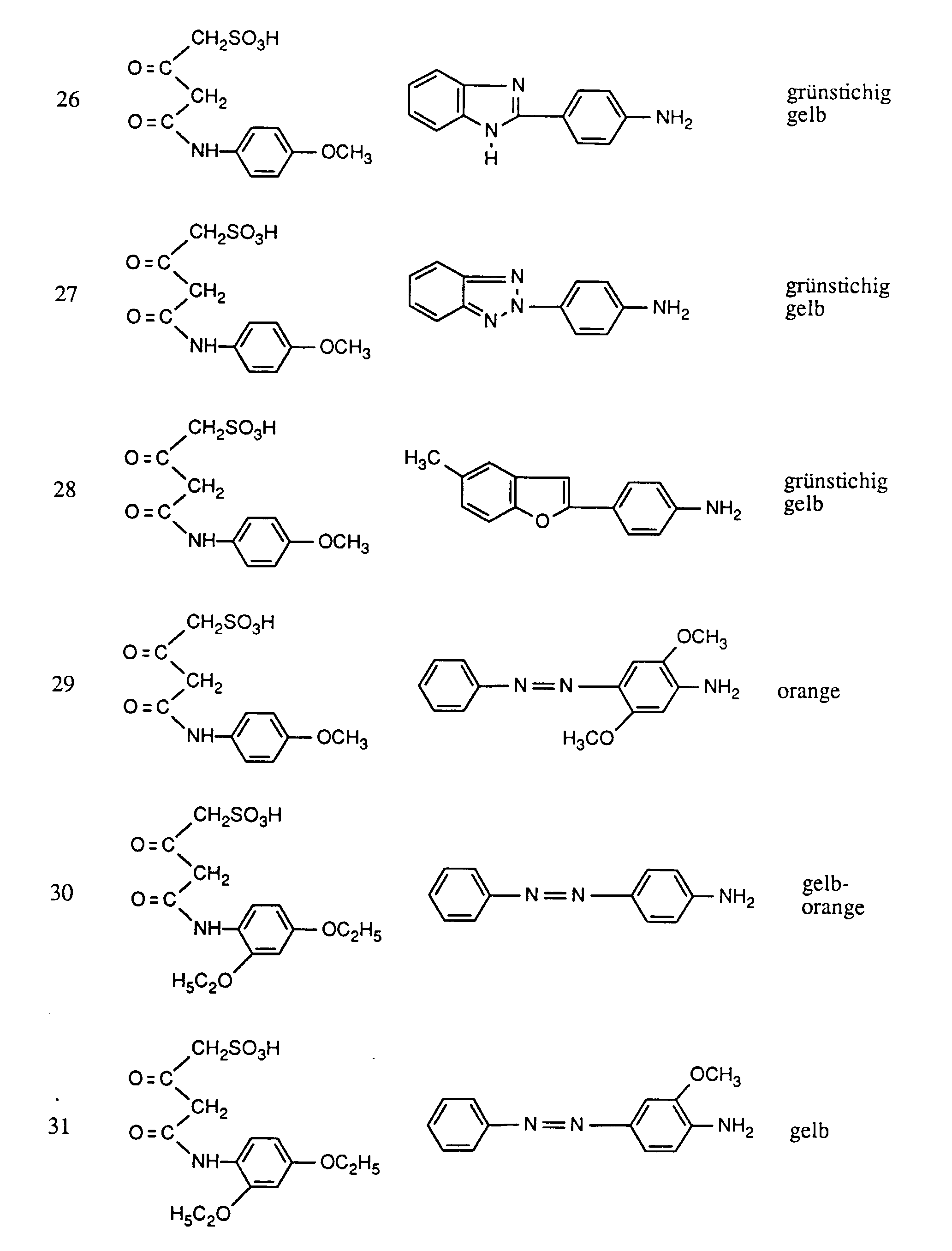

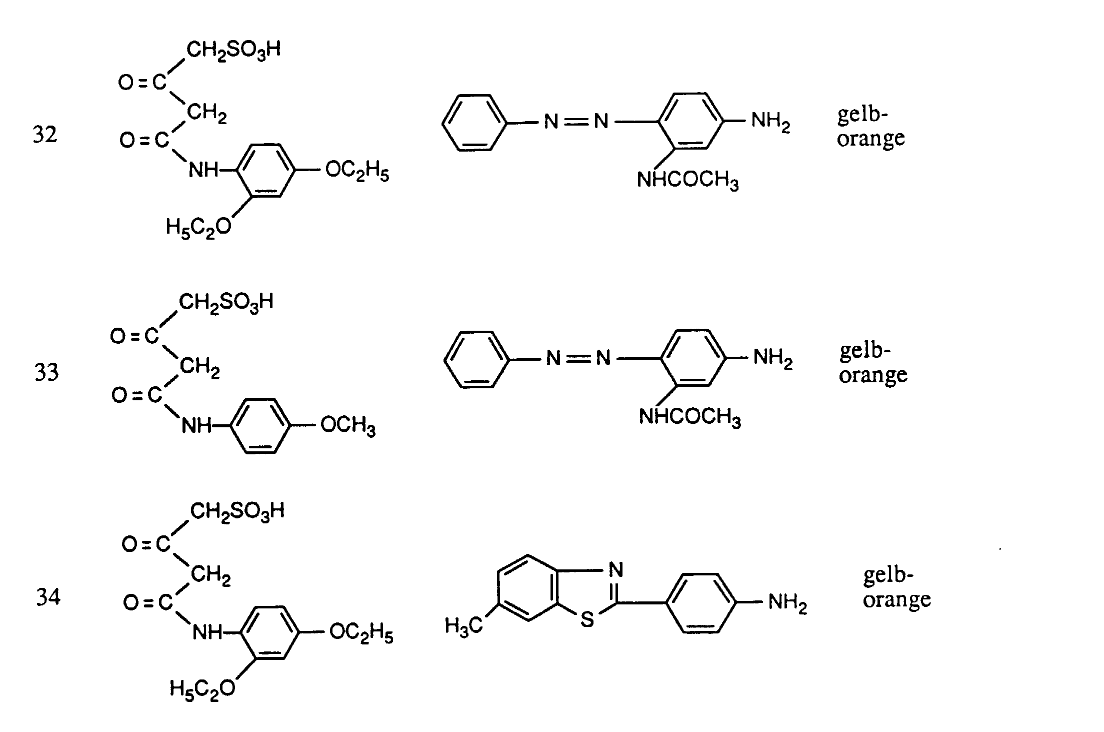

### Färbevorschrift I

Man färbt 10 Teile Polyamid-6,6-Gewebe in 500 Teilen einer wässrigen Motte, die 2 g/l Ammonacetat enthält und mit Essigsäure auf pH 5 gestellt wird. Der Anteil des Farbstoffs der Formel (103) gemäss Beispiel 12 beträgt 0,7 % bezogen auf das Fasergewicht. Die Färbedauer bei einer Temperatur von 98° beträgt 30 bis 90 Minuten. Das gefärbte Polyamid-6,6-Gewebe wird anschliessend herausgenommen und wie üblich gewaschen und getrocknet.

Man erhält ein gelb gefärbtes Polyamid-6,6-Gewebe, das eine reine Nuance und gute Gesamtechtheiten aufweist.

### Färbevorschrift II

Man färbt 10 Teile Polyamid-6,6-Gewebe in 500 Teilen einer wässrigen Flotte, die 1 g/l Mononatriumphosphat enthält und mit Dinatriumphosphat auf pH 6 gestellt wird. Der Anteil des Farbstoffs der Formel (103) gemäss Beispiel 12 beträgt 1 %, bezogen auf das Fasergewicht. Die Färbedauer bei einer Temperatur von 98° beträgt 30 bis 90 Minuten. Das gefärbte Polyamid-6,6-Gewebe wird anschliessend herausgenommen und wie üblich gewaschen und getrocknet.

Man erhält ein gelb gefärbtes Polyamid-6,6-Gewebe, das eine reine Nuance und gute Gesamtechtheiten aufweist.

### Färbevorschrift III

Man färbt 10 Teile Wollstück in 500 Teilen einer wässrigen Flotte. Bezogen auf das Fasergewicht betragen die Anteile des Farbstoffs der Formel (103) gemäss Beispiel 12 0,45%, an Glaubersalz kalz. 5 % und 80%-iger Essigsäure 2 %. Die Färbedauer bei einer Temperatur von 98° beträgt 30-60 Minuten. Das gelb gefärbte, wie üblich gewaschene und getrocknete Wollstück weist sehr gute Allgemeinechtheiten auf.

## Patentansprüche (Patentansprüche für folgende(n) Vertragsstaat(en): BE, CH, DE, FR, GB, IT, LI)

1. Farbstoffe der Formel 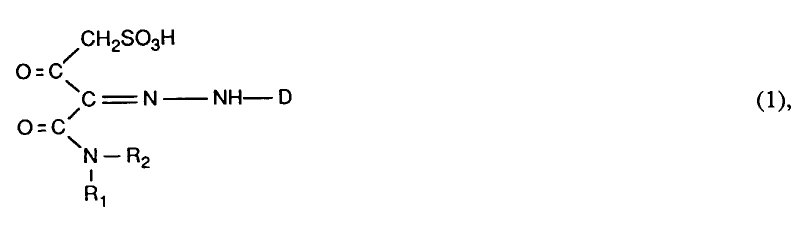 worin D der Rest einer Diazokomponente der Benzol- oder Naphthalinreihe oder der heterocyclischen Reihe ist, R₁ Wasserstoff oder C₁-C₄-Alkyl und R₂ gegebenenfalls substituiertes Phenyl ist.

2. Farbstoffe gemäss Anspruch 1, dadurch gekennzeichnet, dass R₁ Wasserstoff ist.

3. Farbstoffe gemäss einem der Ansprüche 1 und 2, dadurch gekennzeichnet, dass R₂ gegebenenfalls durch C₁-C₄-Alkyl, C₁-C₄-Alkoxy, C₂-C₄-Alkanoylamino, Halogen oder Sulfo substituiertes Phenyl ist.

4. Farbstoffe gemäss einem der Ansprüche 1 bis 3, dadurch gekennzeichnet, dass D ein Rest der Formel 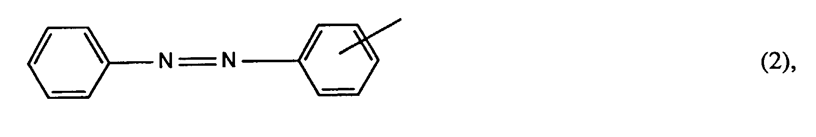 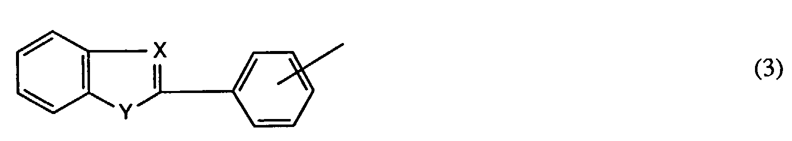 oder 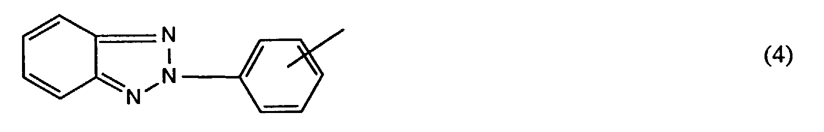 ist, wobei die Reste der Formeln (2), (3) und (4) gegebenenfalls substituiert sind und im Rest der Formel (3) X ein Stickstoffatom oder die CH-Gruppe ist und Y ein Sauerstoffatom, Schwefelatom oder die NH-Gruppe bedeutet.

5. Farbstoffe gemäss einem der Ansprüche 1 bis 4 der Formel 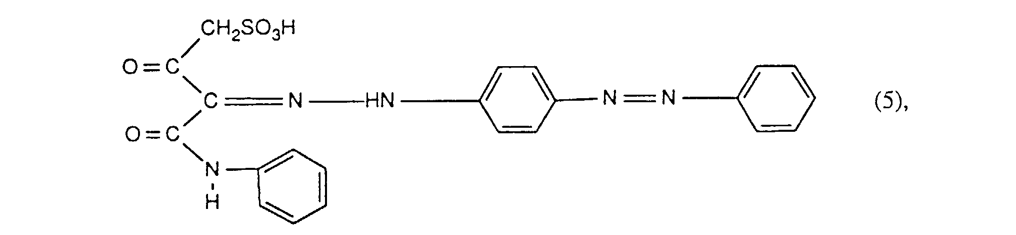 wobei die Farbstoffe der Formel (5) gegebenenfalls durch C₁-C₄-Alkyl, C₁-C₄-Alkoxy, C₂-C₄-Alkanoylamino, Halogen oder Sulfo substituiert sind.

6. Farbstoffe gemäss einem der Ansprüche 1 bis 4 der Formeln 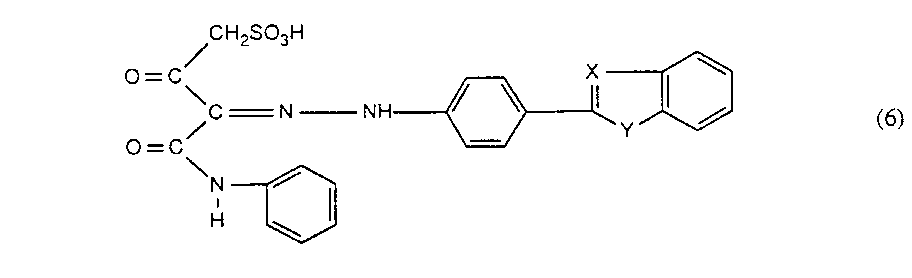 und 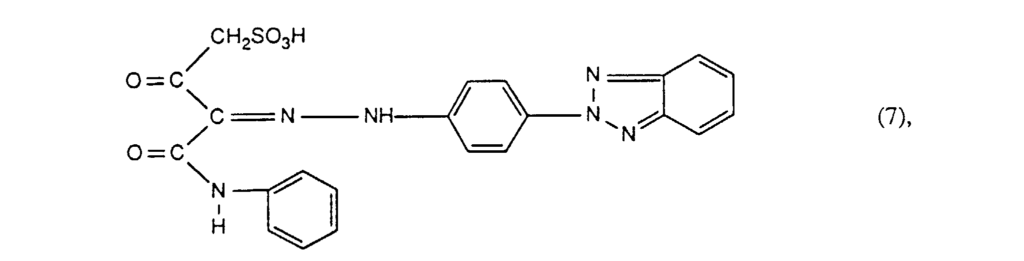 wobei die Farbstoffe der Formeln (6) und (7) gegebenenfalls durch C₁-C₄-Alkyl, C₁-C₄-Alkoxy, C₂-C₄-Alkanoylamino, Halogen oder Sulfo substituiert sind und im Farbstoff der Formel (6) X ein Stickstoffatom oder die CH-Gruppe ist und Y ein Sauerstoffatom, Schwefelatom oder die NH-Gruppe bedeutet.

7. Verfahren zur Herstellung von Farbstoffen gemäss Anspruch 1, dadurch gekennzeichnet, dass man ein Amin der Formel
D-NH₂ (8)
diazotiert und auf eine Kupplungskomponente der Formel 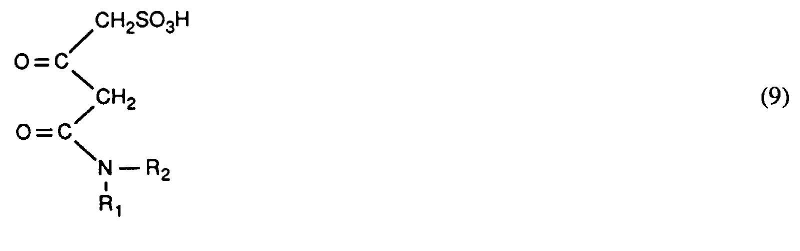 kuppelt, wobei D, R₁ und R₂ die in Anspruch 1 angegebenen Bedeutungen haben

8. Verwendung der Farbstoffe gemäss den Ansprüchen 1 bis 6 bzw. der gemäss Anspruch 7 erhaltenen Farbstoffe zum Färben und Bedrucken von stickstoffhaltigen oder hydroxygruppenhaltigen Fasermaterialien, Papier oder Leder.

9. Verwendung gemäss Anspruch 8 zum Färben und Bedrucken von natürlichem und synthetischem Polyamidfasermaterial.

10. Verfahren zum Trichromie-Färben oder -Bedrucken von natürlichem und synthetischem Polyamidfasermaterial mit Farbstoffmischungen, dadurch gekennzeichnet, dass man einen Farbstoff gemäss Anspruch 1 zusammen mit einem rotfärbenden Farbstoff und einem blaufärbenden Farbstoff verwendet.

11. Verbindungen der Formel 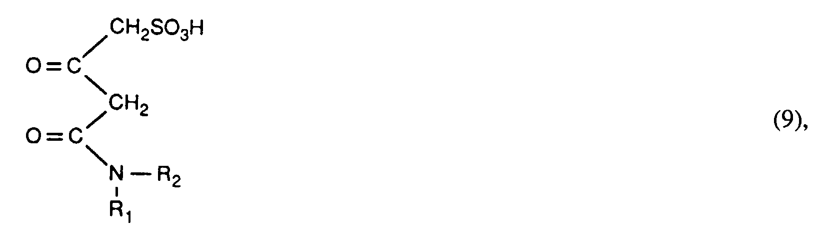 worin R₁ Wasserstoff oder C₁-C₄-Alkyl und R₂ gegebenenfalls substituiertes Phenyl ist.

## Patentansprüche (Patentansprüche für folgende(n) Vertragsstaat(en): ES)

1. Verfahren zur Herstellung von Farbstoffen der Formel 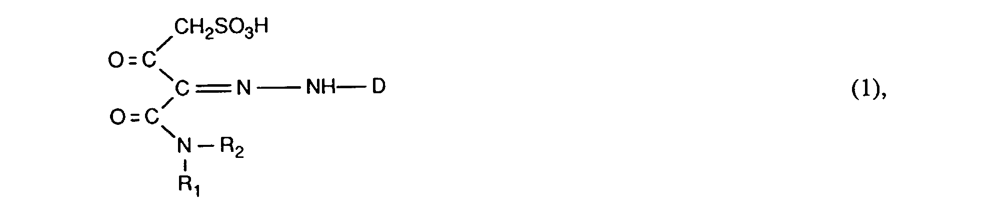 worin D der Rest einer Diazokomponente der Benzol- oder Naphthalinreihe oder der heterocyclischen Reihe ist, R₁ Wasserstoff oder C₁-C₄-Alkyl und R₂ gegebenenfalls substituiertes Phenyl ist, dadurch gekennzeichnet, dass man ein Amin der Formel
D-NH₂ (8)
diazotiert und auf eine Kupplungskomponente der Formel 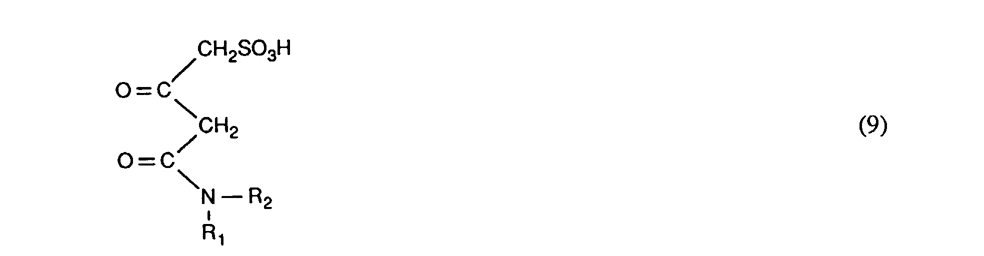 kuppelt, wobei D, R₁ und R₂ die unter Formel (1) angegebenen Bedeutungen haben.

2. Verfahren gemäss Anspruch 1, dadurch gekennzeichnet, dass R₁ Wasserstoff ist.

3. Verfahren gemäss einem der Ansprüche 1 und 2, dadurch gekennzeichnet, dass R₂ gegebenenfalls durch C₁-C₄-Alkyl, C₁-C₄-Alkoxy, C₂-C₄-Alkanoylamino, Halogen oder Sulfo substituiertes Phenyl ist.

4. Verfahren gemäss einem der Ansprüche 1 bis 3, dadurch gekennzeichnet, dass D ein Rest der Formel 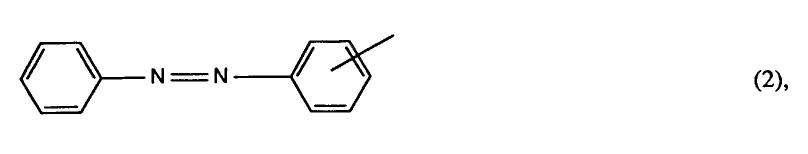 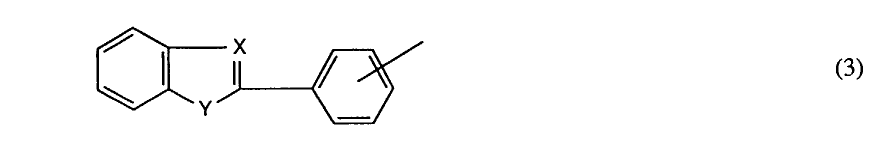 oder 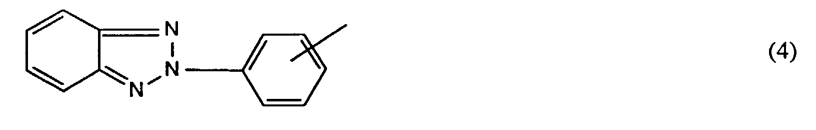 ist, wobei die Reste der Formeln (2), (3) und (4) gegebenenfalls substituiert sind und im Rest der Formel (3) X ein Stickstoffatom oder die CH-Gruppe ist und Y ein Sauerstoffatom, Schwefelatom oder die NH-Gruppe bedeutet.

5. Verfahren gemäss einem der Ansprüche 1 bis 4 zur Herstellung von Farbstoffen der Formel 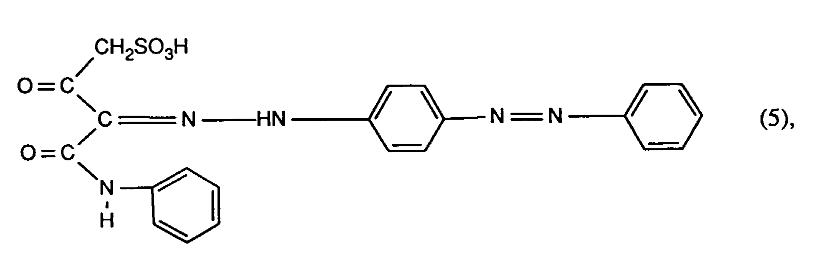 wobei die Farbstoffe der Formel (5) gegebenenfalls durch C₁-C₄-Alkyl, C₁-C₄-Alkoxy, C₂-C₄-Alkanoylamino, Halogen oder Sulfo substituiert sind.

6. Verfahren gemäss einem der Ansprüche 1 bis 4 zur Herstellung von Farbstoffen der Formeln 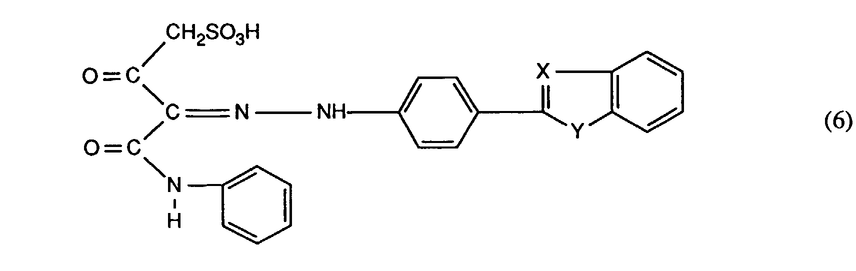 und 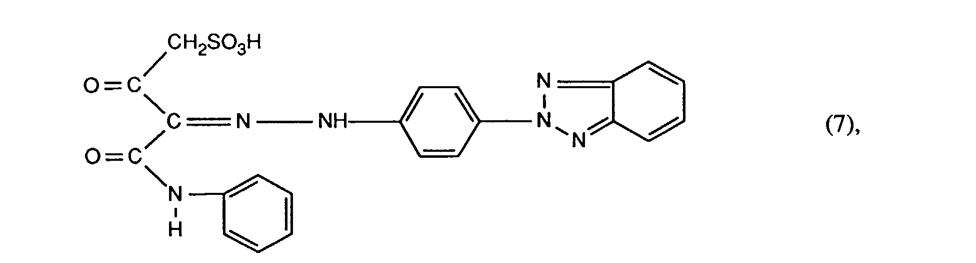 wobei die Farbstoffe der Formeln (6) und (7) gegebenenfalls durch C₁-C₄-Alkyl, C₁-C₄-Alkoxy, C₂-C₄-Alkanoylamino, Halogen oder Sulfo substituiert sind und im Farbstoff der Formel (6) X ein Stickstoffatom oder die CH-Gruppe ist und Y ein Sauerstoffatom, Schwefelatom oder die NH-Gruppe bedeutet.

7. Verwendung der gemäss den Ansprüchen 1 bis 6 erhaltenen Farbstoffe zum Färben und Bedrucken von stickstoffhaltigen oder hydroxygruppenhaltigen Fasermaterialien, Papier oder Leder.

8. Verwendung gemäss Anspruch 7 zum Färben und Bedrucken von natürlichem und synthetischem Polyamidfasermaterial.

9. Verfahren zum Trichromie-Färben oder -Bedrucken von natürlichem und synthetischem Polyamidfasermaterial mit Farbstoffmischungen, dadurch gekennzeichnet, dass man einen gemäss Anspruch 1 erhaltenen Farbstoff zusammen mit einem rotfärbenden Farbstoff und einem blaufärbenden Farbstoff verwendet

10. Verfahren zur Herstellung von Verbindungen der Formel 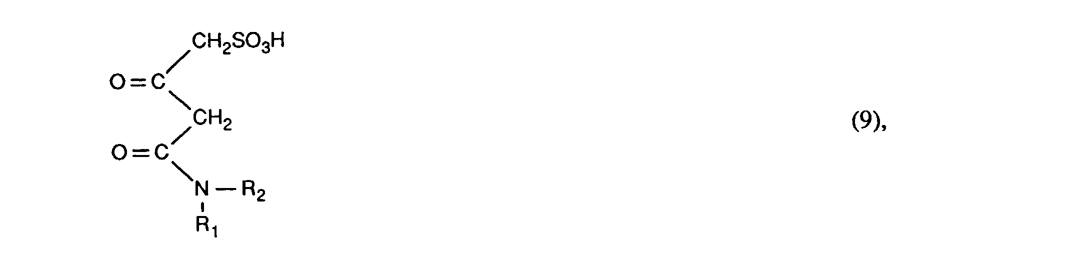 worin R₁ Wasserstoff oder C₁-C₄-Alkyl und R₂ gegebenenfalls substituiertes Phenyl ist, dadurch gekennzeichnet, dass man eine Verbindung der Formel 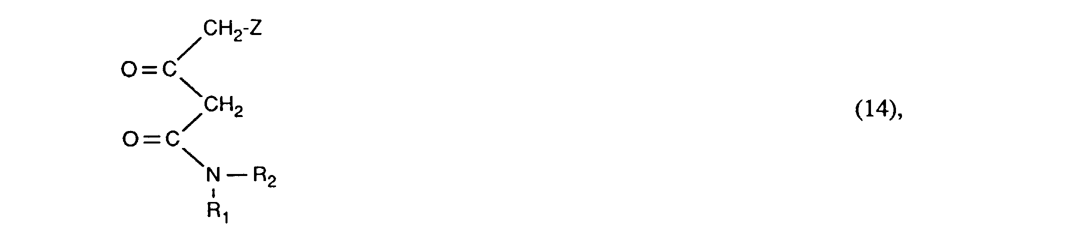 worin R₁ und R₂ die unter Formel (9) angegebenen Bedeutungen haben und Z eine anionische Abgangsgruppe ist, durch Austausch des Restes Z durch eine Sulfogruppe zur Verbindung der Formel (9) umsetzt.

## Claims (Claims for the following Contracting State(s): BE, CH, DE, FR, GB, IT, LI)

1. A dye of formula 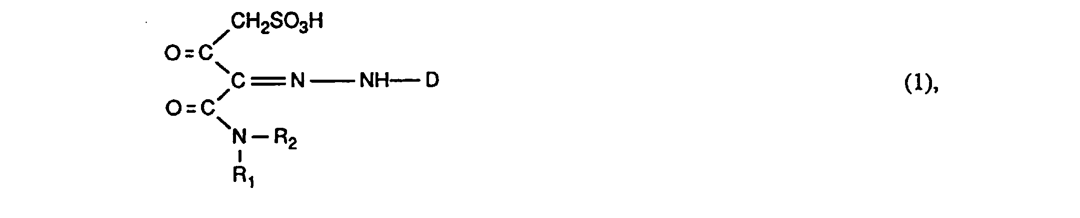 wherein D is the radical of a diazo component of the benzene or naphthalene series or of the heterocyclic series, R₁ is hydrogen or C₁-C₄alkyl and R₂ is phenyl or substituted phenyl.

2. A dye according to claim 1, wherein R₁ is hydrogen.

3. A dye according to either claim 1 or claim 2, wherein R₂ is phenyl or phenyl which is substituted by C₁-C₄alkyl, C₁-C₄alkoxy, C₂-C₄alkanoylamino, halogen or sulfo.

4. A dye according to any one of claims 1 to 3, wherein D is a radical of formula 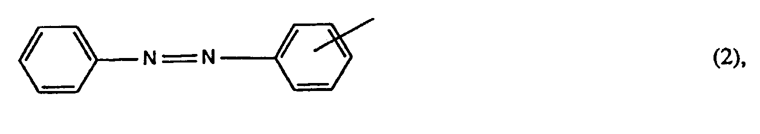 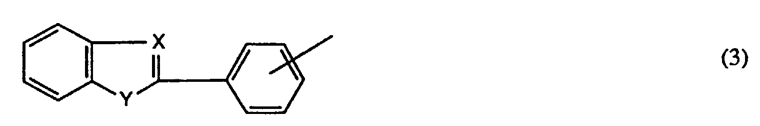 or 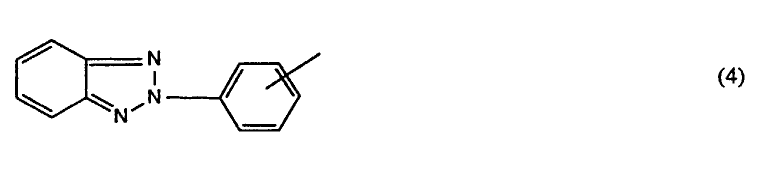 which radical of formula (2), (3) or (4) is unsubstituted or substituted and, in the radical of formula (3), X is a nitrogen atom or the CH group and Y is an oxygen atom, a sulfur atom or the NH group.

5. A dye according to any one of claims 1 to 4 of formula 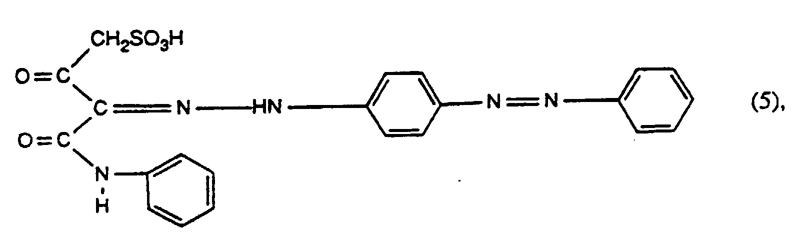 which dye of formula (5) may be substituted by C₁-C₄alkyl, C₁-C₄alkoxy, C₂-C₄alkanoylamino, halogen or sulfo.

6. A dye according to any one of claims 1 to 4 of formula 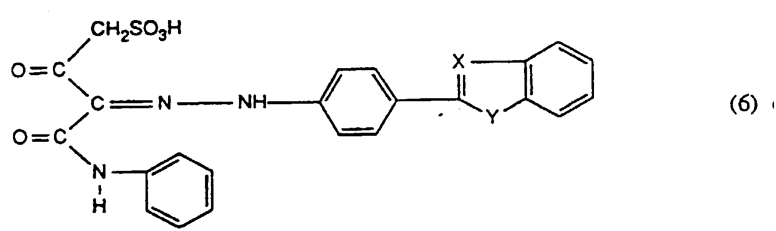 or 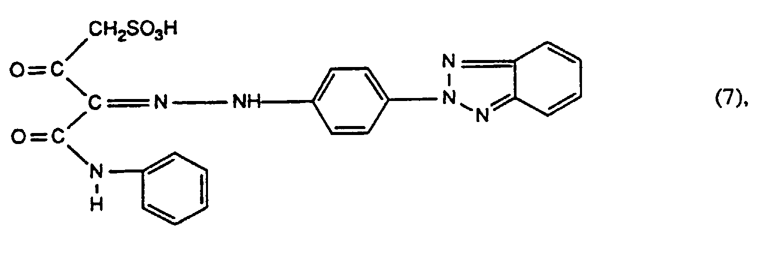 which dye of formula (6) or (7) may be substituted by C₁-C₄alkyl, C₁-C₄alkoxy, C₂-C₄alkanoylamino, halogen or sulfo, and, in the dye of formula (6), X is a nitrogen atom or the CH group and Y is an oxygen atom, a sulfur atom or the NH group.

7. A process for the preparation of a dye according to claim 1, which comprises diazotising an amine of formula
D-NH₂ (8)
and coupling the diazotised amine to a coupling component of formula 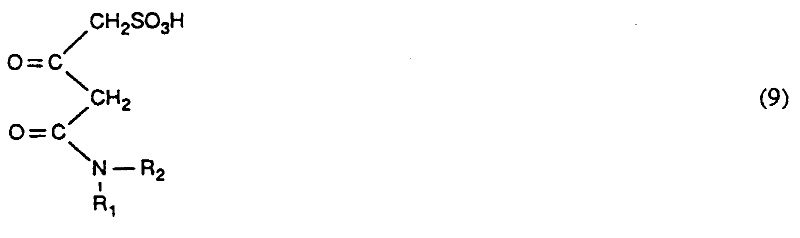 wherein D, R₁ and R₂ are as defined in claim 1.

8. Use of a dye according to claims 1 to 6 or of a dye obtained according to claim 7 for dyeing or printing nitrogen-containing or hydroxyl-containing fibre materials, paper or leather.

9. Use according to claim 8 for dyeing or printing natural and synthetic polyamide fibre material.

10. A process for the trichromatic dyeing or printing of natural and synthetic polyamide fibre material with dye mixtures, which comprises using a dye according to claim 1 together with a red-dyeing dye and a blue-dyeing dye.

11. A compound of formula 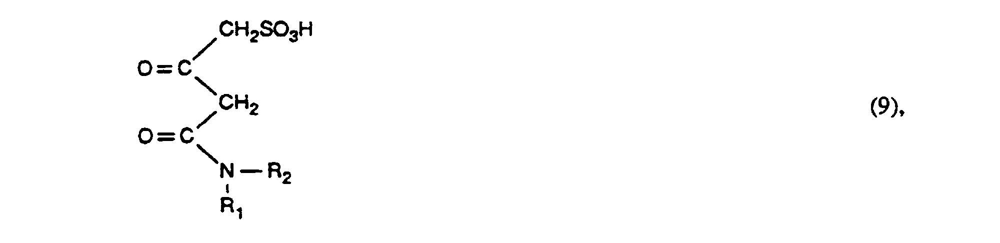 wherein R₁ is hydrogen or C₁-C₄alkyl and R₂ is phenyl or substituted phenyl.

## Claims (Claims for the following Contracting State(s): ES)

1. A process for the preparation of a dye of formula 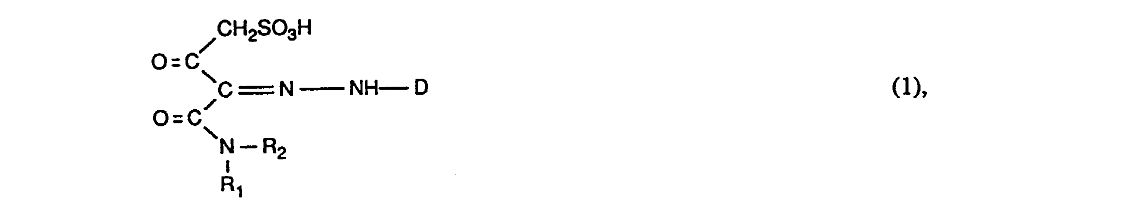 wherein D is the radical of a diazo component of the benzene or naphthalene series or of the heterocyclic series, R₁ is hydrogen or C₁-C₄alkyl and R₂ is phenyl or substituted phenyl, which comprises diazotising an amine of formula
D-NH₂ (8)
and coupling the diazotised amine to a coupling component of formula 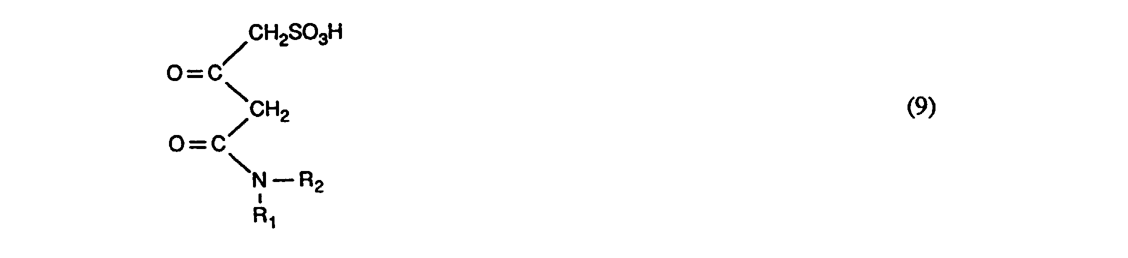 wherein D, R₁ and R₂ are as defined under formula (1).

2. A process according to claim 1, wherein R₁ is hydrogen.

3. A process according to either claim 1 or claim 2, wherein R₂ is phenyl or phenyl which is substituted by C₁-C₄alkyl, C₁-C₄alkoxy, C₂-C₄alkanoylamino, halogen or sulfo.

4. A process according to any one of claims 1 to 3, wherein D is a radical of formula 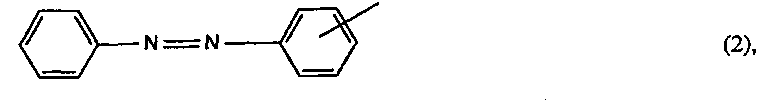 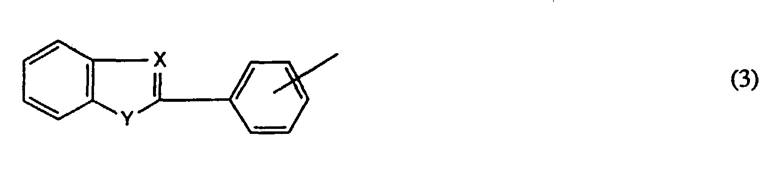 or 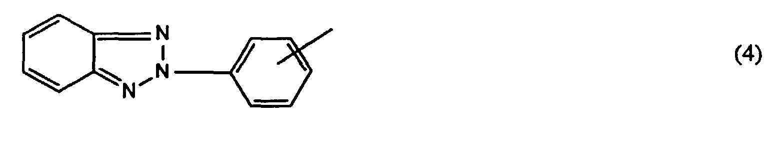 which radical of formula (2), (3) or (4) is unsubstituted or substituted and, in the radical of formula (3), X is a nitrogen atom or the CH group and Y is an oxygen atom, a sulfur atom or the NH group.

5. A process according to any one of claims 1 to 4 for the preparation of a dye of formula 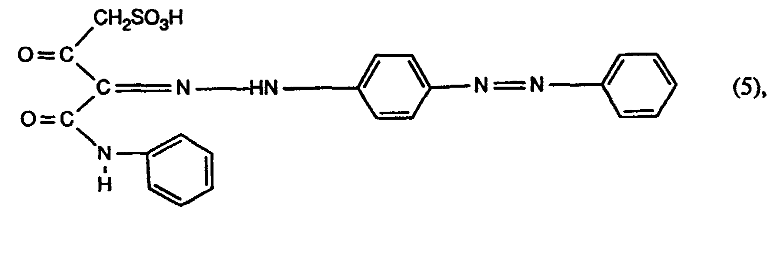 which dye of formula (5) may be substituted by C₁-C₄alkyl, C₁-C₄alkoxy, C₂-C₄alkanoylamino, halogen or sulfo.

6. A process according to any one of claims 1 to 4 for the preparation of a dye of formula 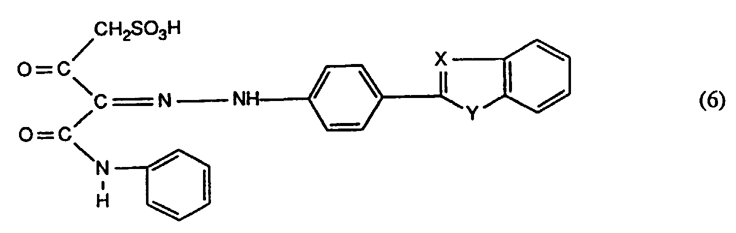 or 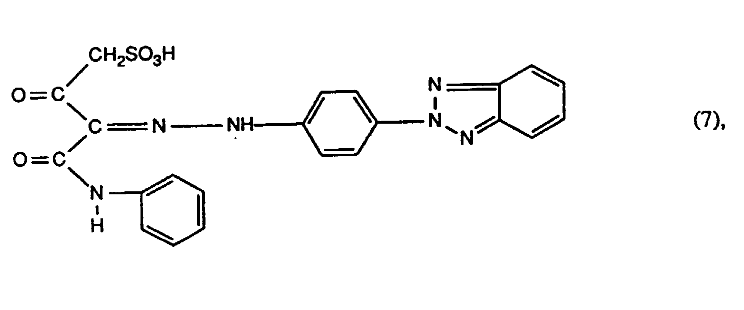 which dye of formula (6) or (7) may be substituted by C₁-C₄alkyl, C₁-C₄alkoxy, C₂-C₄alkanoylamino, halogen or sulfo, and, in the dye of formula (6), X is a nitrogen atom or the CH group and Y is an oxygen atom, a sulfur atom or the NH group.

7. Use of a dye obtained according to claims 1 to 6 for dyeing or printing nitrogen-containing or hydroxyl-containing fibre materials, paper or leather.

8. Use according to claim 7 for dyeing or printing natural and synthetic polyamide fibre material.

9. A process for the trichromatic dyeing or printing of natural and synthetic polyamide fibre material with dye mixtures, which comprises using a dye obtained according to claim 1 together with a red-dyeing dye and a blue-dyeing dye.

10. A process for the preparation of a compound of formula 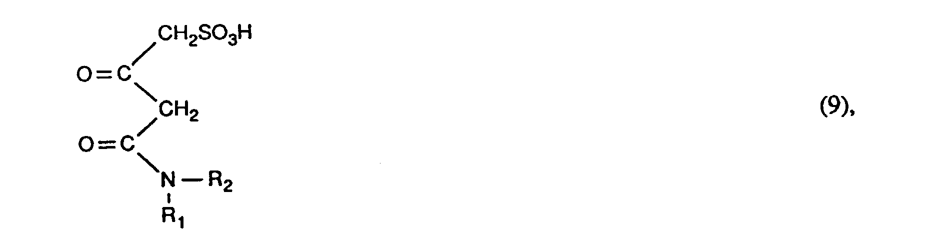 wherein R₁ is hydrogen or C₁-C₄alkyl and R₂ is phenyl or substituted phenyl, which comprises reacting a compound of formula 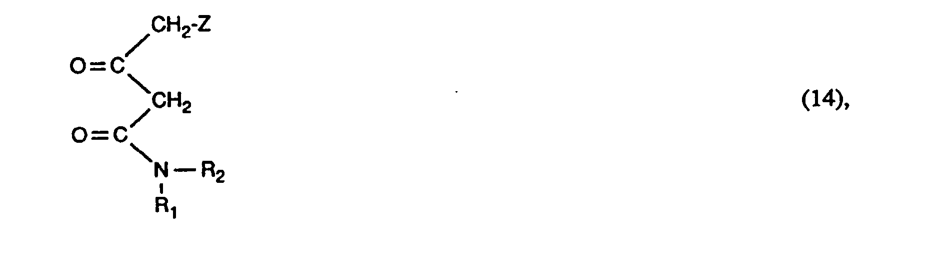 wherein R₁ and R₂ are as defined under formula (9) and Z is an anionic leaving group, by exchange of the radical Z for a sulfo group, to give the compound of formula (9).

## Revendications (Revendications pour l'(les) Etat(s) contractant(s) suivant(s): BE, CH, LI, DE, FR, GB, IT)

1. Colorants de formule 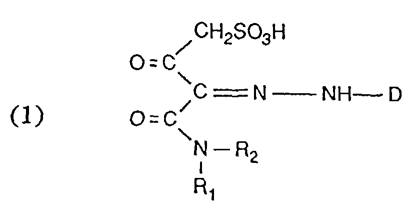 dans laquelle D représente le résidu d'un composant diazo de la série benzénique ou naphtalénique ou de la série hétérocyclique, R₁ représente un atome d'hydrogène ou un résidu alkyle en C₁₋₄ et R₂ un résidu phényle pouvant être substitué.

2. Colorants conformes à la revendication 1, caractérisés en ce que R₁ est un atome d'hydrogène.

3. Colorants conformes à une des revendications 1 et 2, caractérisés en ce que R₂ représente un noyau phényle pouvant être substitué par un groupe alkyle en C₁₋₄, alcoxy en C₁₋₄, alcanoylamino en C₂₋₄, halogéno ou sulfo.

4. Colorants conformes à une des revendications 1 à 3, caractérisés en ce que D est un résidu de formule 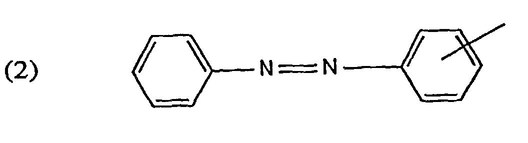 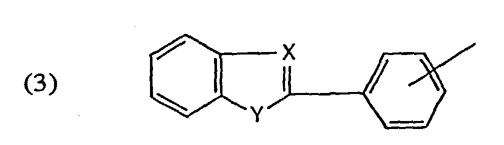 ou 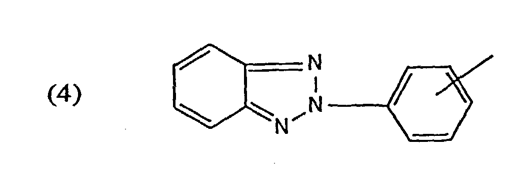 où les résidus de formule (2), (3) et (4) peuvent être substitués, et, dans le résidu de formule (3), X représente un atome d'azote ou un groupe CH et Y est un atome d'oxygène, un atome de soufre ou un groupe NH.

5. Colorants conformes à une des revendications 1 à 4 de formule 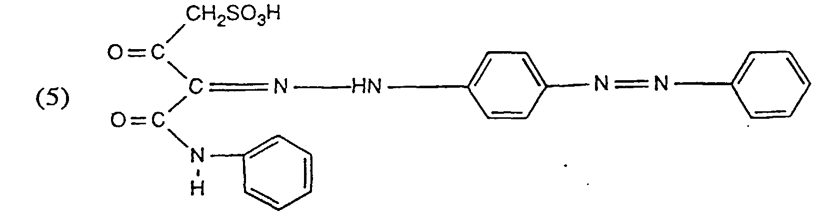 où les colorants de formule (5) peuvent être substitués par des groupes alkyle en C₁₋₄, alcoxy en C₁₋₄, halogéno, alcanoylamino en C₂₋₄ ou sulfo.

6. Colorants conformes à une des revendications 1 à 4 de formule 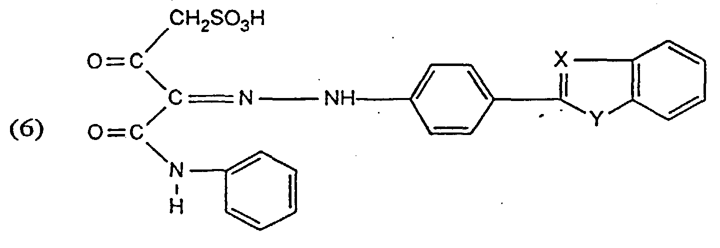 et 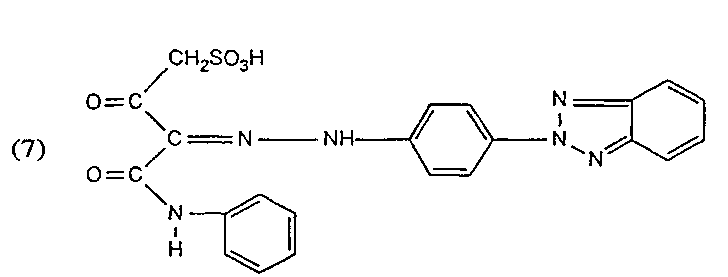 où les colorants de formule (6) et (7) peuvent porter des groupes alkyle en C₁₋₄, alcoxy en C₁₋₄, alcanoylamino en C₂₋₄, halogéno ou sulfo et, dans le colorant de formule (6), X représente un atome d'azote ou un groupe CH et Y représente un atome d'oxygène, un atome de soufre ou un groupe NH.

7. Procédé pour la préparation des colorants conformes à la revendication 1, lequel procédé est caractérisé en ce que l'on soumet une amine de formule
(8) D-NH₂
à une diazotation et en ce que l'on copule l'amine diazotée avec un copulant de formule 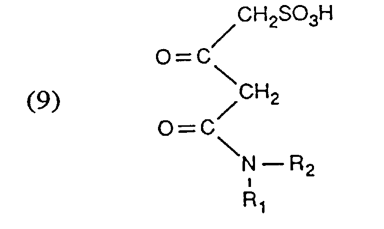 D, R₁ et R₂ ayant la signification indiquée dans la revendication 1.

8. Utilisation des colorants conformes aux revendications 1 à 6 ou obtenus conformément à la revendication 7 pour la teinture ou l'impression de matériaux fibreux contenant des atomes d'azote ou des groupes hydroxyle, du papier ou du cuir.

9. Utilisation conforme à la revendication 8 pour la teinture et l'impression de matériaux fibreux en polyamide naturel ou synthétique.

10. Procédé de teinture ou d'impression en trichromie de matériaux fibreux en polyamide naturel ou synthétique avec des mélanges de colorants, caractérisé en ce que l'on utilise un colorant conforme à la revendication 1 en combinaison avec un colorant rouge et un colorant bleu.

11. Composés de formule 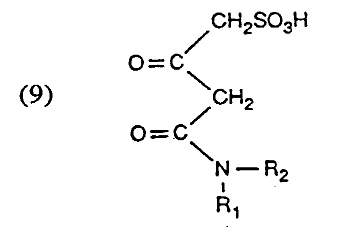 où R₁ représente un atome d'hydrogène ou un groupe alkyle en C₁₋₄ et R₂ un groupe phényle pouvant être substitué.

## Revendications (Revendications pour l'(les) Etat(s) contractant(s) suivant(s): ES)

1. Procédé pour la préparation de colorants de formule 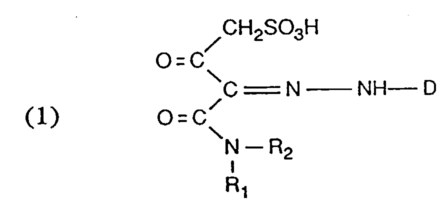 dans laquelle D représente le résidu d'un composant diazo de la série benzénique ou naphtalénique ou de la série hétérocyclique, R₁ représente un atome d'hydrogène ou un résidu alkyle en C₁₋₄ et R₂ un résidu phényle pouvant être substitué, lequel procédé est caractérisé en ce que l'on soumet une amine de formule
(8) D-NH₂
à une diazotation et en ce que l'on copule l'amine diazotée avec un copulant de formule 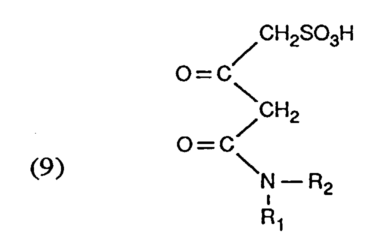 D, R₁ et R₂ ayant la signification indiquée pour la formule (1).

2. Procédé conforme à la revendication 1, caractérisé en ce que R₁ est un atome d'hydrogène.

3. Procédé conforme à une des revendications 1 et 2, caractérisé en ce que R₂ représente un noyau phényle pouvant être substitué par un groupe alkyle en C₁₋₄, alcoxy en C₁₋₄, alcanoylamino en C₂₋₄, halogéno ou sulfo.

4. Procédé conforme à une des revendications 1 à 3, caractérisé en ce que D est un résidu de formule 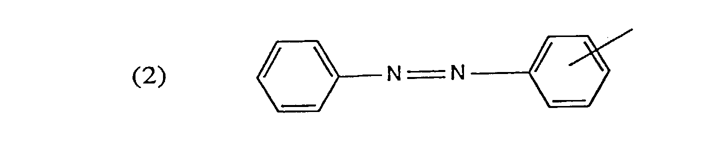 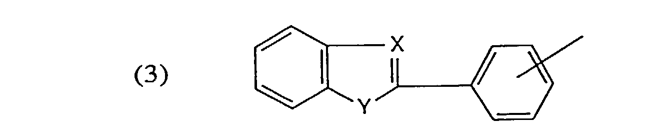 ou 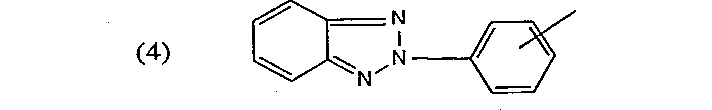 où les résidus de formule (2), (3) et (4) peuvent être substitués, et, dans le résidu de formule (3), X représente un atome d'azote ou un groupe CH et Y est un atome d'oxygène, un atome de soufre ou un groupe NH.

5. Procédé conforme à une des revendications 1 à 4 pour la préparation de colorants de formule 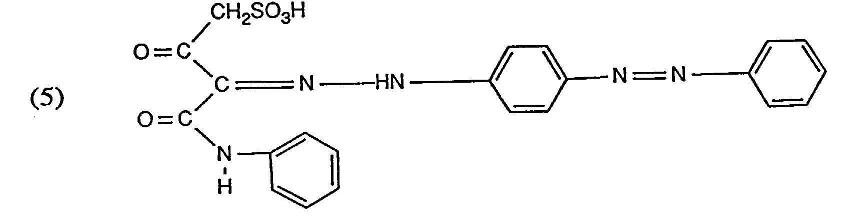 les colorants de formule (5) pouvant être substitués par des groupes alkyle en C₁₋₄, alcoxy en C₁₋₄, halogéno, alcanoylamino en C₂₋₄ ou sulfo.

6. Procédé conforme à une des revendications 1 à 4 pour la préparation de colorants de formule 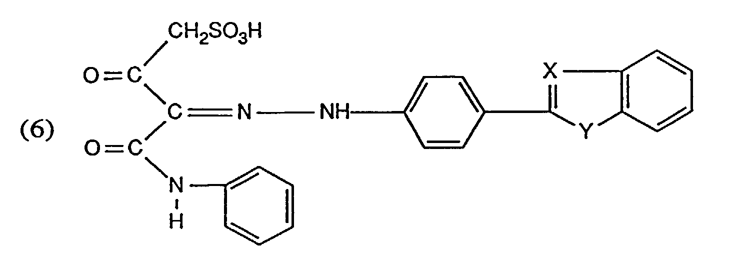 et 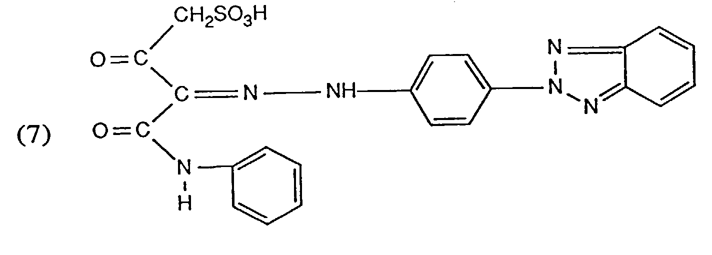 où les colorants de formule (6) et (7) peuvent porter des groupes alkyle en C₁₋₄, alcoxy en C₁₋₄, alcanoylamino en C₂₋₄, halogéno ou sulfo et, dans le colorant de formule (6), X représente un atome d'azote ou un groupe CH et Y représente un atome d'oxygène, un atome de soufre ou un groupe NH.

7. Utilisation des colorants obtenus conformément aux revendications 1 à 6 pour la teinture ou l'impression de matériaux fibreux contenant des atomes d'azote ou des groupes hydroxyle, du papier ou du cuir.

8. Utilisation conforme à la revendication 7 pour la teinture et l'impression de matériaux fibreux en polyamide naturel ou synthétique.

9. Procédé de teinture ou d'impression en trichromie de matériaux fibreux en polyamide naturel ou synthétique avec des mélanges de colorants, caractérisé en ce que l'on utilise un colorant obtenus conformément à la revendication 1, en combinaison avec un colorant rouge et un colorant bleu.

10. Procédé pour la préparation de composés de formule 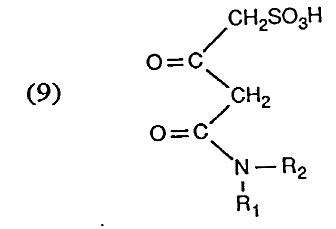 dans laquelle R₁ représente un atome d'hydrogène ou un groupe alkyle en C₁₋₄ et R₂ un groupe phényle pouvant être substitué, caractérisé en ce que l'on remplace, dans un composé de formule 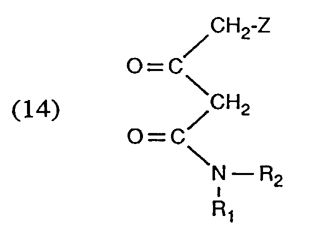 dans laquelle R₁ et R₂ ont la signification indiquée pour la formule (9) et Z est un groupe partant anionique, le résidu Z par un groupe sulfo ce qui donne un composé de formule (9).
